# EUROPÄISCHE PATENTSCHRIFT

(11) **EP 2 628 497 B1**
(45) Veröffentlichungstag und Bekanntmachung des Hinweises auf die Patenterteilung: **12.10.2016**
(21) Anmeldenummer: 13167921.9
(22) Anmeldetag: 30.03.2010
(51) Int. Cl.: A61M 16/00

(54) **Beatmungs- und Anästhesiesystem**
Ventilation and anaesthetic system
Système de respiration et d'anesthésie

(43) Veröffentlichungstag der Anmeldung: 21.08.2013
(62) Teilanmeldung aus: 10158480.3
(73) Patentinhaber: Drägerwerk AG & Co. KGaA, 23558 Lübeck (DE)
(72) Erfinder: Hansmann, Hans-Ulrich, 23858 Bamitz (DE); Eger, Marcus, 23562 Lübeck (DE); Glaw, Tobias, 23558 Lübeck (DE); Krüger, Thomas, 23858 Reinfeld (DE)

(56) Entgegenhaltungen:
- WO-A1-2006/131149
- AU-B2- 707 148
- DE-A1- 10 164 446
- DE-B3-102007 062 214
- US-A- 5 353 788
- US-A1- 2006 152 197
- US-A1- 2007 163 588

## Beschreibung

Die vorliegende Erfindung betrifft eine Beatmungs- oder Anästhesiesystem gemäß dem Oberbegriff des Anspruches 1.

Für verschiedene medizinische Anwendungen, z. B. bei Operationen, ist eine Beatmung von Patienten notwendig. Beatmungsgeräte bzw. Beatmungssysteme dienen zur Beatmung von Patienten und können zusätzlichen mit einem Anästhesiemittelreflektor und Anästhesiemitteldosierer als Anästhesiegeräte bzw. Anästhesiesysteme auch zur Narkose eingesetzt werden. Bei einigen Beatmungs-oder Anästhesiesystemen kann wenigstens teilweise das von dem Patienten ausgeatmete Exspirationsgas wieder als Inspirationsgas wiederverwendet werden, d. h. es handelt sich um ein Rückatemsystem mit einem Atemluftkreissystem. In dem Beatmungsgerät mit dem Atemluftkreissystem ist eine Gasfördereinrichtung vorhanden, welche die Atemluft während der Inspiration zum Patienten leitet. Während und nach der Ausatmung ist die Gasfördereinrichtung entweder abgeschaltet oder wird nur mit einem sehr geringen Förderstrom betrieben.

Die von dem Beatmungs- oder Anästhesiesystem durchgeführte Beatmung kann entweder vollständig die Eigenbeatmungstätigkeit des Patienten übernehmen oder bei einem unterstützenden Verfahren die eigenen Atemtätigkeit des Patienten lediglich nur teilweise ersetzten, so dass die Atemmuskulatur des Patienten einen Teil der Atemtätigkeit übernimmt. Für eine verbesserte Therapie, insbesondere bei COPD-Patienten, ist eine Steuerung des Anteils der Beatmung erforderlich, d. h. der Anteil der Eigenbeatmungstätigkeit des Patienten kann gesteuert und/oder geregelt werden. Hierzu sind die Leistung der Gasfördereinheit des Beatmungs- oder Anästhesiesystems zu steuern und der Zeitpunkt und die Amplitude der Beatmung in Bezug auf die der Eigenatmung des Patienten anzupassen. Dazu wird mit elektromyographischen Sensoren (EMG-Sensoren) invasiv die elektromyographische Aktivität der Atemmuskulatur des Patienten erfasst. Die EMG-Sensoren sind invasiv, insbesondere in einer Luftröhre, am Patienten angeordnet.

Die DE 10 2007 062 214 B3 zeigt ein Verfahren zum automatischen Steuern eines Beatmungssystems für proportional unterstützende Ventilation mit einer Steuereinrichtung und einem Ventilator, der ein Atemgas mit einem von der Steuereinheit vorgegebenen Druck liefert. Mit Elektroden wird elektromyographisch ein elektrisches Signal aufgenommen und hieraus die Atemaktivität und der Atemmuskeldruck bestimmt.

Das Dokument WO 2006/131149 A1 offenbart ein Beatmungs- oder Anästhesiesystem zur Beatmung eines Patienten, umfassend eine Gasfördereinrichtung, wenigstens eine Gasleitung zur Ausbildung eines Atemluftleitungssystems, wenigstens ein EMG-Sensor geeignet zur nicht-invasiven Erfassung der elektromyografischen Muskelaktivität der Atemmuskulatur eines zu beatmenden Patienten an dessen Hautoberfläche, eine Steuereinrichtung zur Steuerung und/oder Regelung der Gasfördereinrichtung in Abhängigkeit von der erfassten Muskelaktivität der Atemmuskulatur, eine Haupteinheit, welche die Gasfördereinrichtung aufweist, und wenigstens ein Messmodul, an welchem der wenigstens eine EMG-Sensor angeordnet ist.

Das Dokument US 2007/163588A1 offenbart ein Beatmungsgerät, welches als Stromversorgung eine Batterie mit einem integrierten Ladegerät aufweist. Hierbei können zwei Energieversorgungseinheiten in entsprechenden Aufnahmevorrichtungen in einer Haupteinheit vorgesehen werden. Sowohl eine wiederaufladbare Batterie als auch eine wiederaufladbare Batteriepackung sind vorgesehen. Diese wiederaufladbaren Batterien können durch ein Ladegerät aufgeladen werden. Das Ladegerät kann permanent mit diesen Energiespeichern verbunden und hierzu in einer Aufnahmevorrichtung der Haupteinheit vorgesehen sein.

Die Aufgabe der vorliegenden Erfindung besteht deshalb darin, ein Beatmungs- oder Anästhesiesystem und ein Verfahren zur Steuerung und/oder Regelung eines Beatmungs- oder Anästhesiesystems zur Verfügung zu stellen, bei dem eine Anpassung des Anteils der Beatmung an die Leistungsfähigkeit der Atemmuskulatur des beatmeten Patienten ohne eine invasive Messung der elektromyographischen Aktivität der Atemmuskulatur möglich ist.

Diese Aufgabe wird gelöst mit einem Beatmungs- oder Anästhesiesystem zur Beatmung eines Patienten, umfassend eine Gasfördereinrichtung, wenigstens eine Gasleitung zur Ausbildung eines Atemluftleitungssystems, insbesondere eines Atemluftkreissystems, wenigstens einen EMG-Sensor (elektromyographischer Sensor) zur nicht-invasiven Erfassung der elektromyographischen Muskelaktivität der Atemmuskulatur eines zu beatmenden Patienten an dessen Hautoberfläche, eine Steuereinrichtung zur Steuerung und/oder Regelung der Leistung der Gasfördereinrichtung in Abhängigkeit von der erfassten Muskelaktivität der Atemmuskulatur, eine Haupteinheit mit einem Hauptgehäuse und der Gasfördereinrichtung, wenigstens ein Messmodul, an welchem der wenigstens eine EMG-Sensor angeordnet ist, wobei das wenigstens eine Messmodul eine von der Haupteinheit getrennte Baueinheit ist, dadurch gekennzeichnet, dass die Haupteinheit eine Ladungs- und Schalteinheit umfasst, wobei in der Ladungs- und Schalteinheit Batterien mittels Ladungsteilen aufgeladen werden, dass die Ladungs- und Schalteinheit wiederum Schalteinheiten umfasst, welche dazu ausgebildet sind, nur eine erste Batterie mit dem Messmodul zur Stromversorgung des Messmoduls zu verbinden, ferner wenigstens eine andere Batterie nur mit einem Ladungsteil zum Aufladen der anderen Batterie zu verbinden, und ferner die erste Batterie nach dem Entladen von dem Messmodul elektrisch zu trennen und die andere, aufgeladene Batterie mit dem Messmodul elektrisch zu verbinden, wobei der wenigstens eine EMG-Sensor ein sEMG-Sensor ist. Der sEMG-Sensor ist damit ein surface EMG-Sensor und erfasst die elektrische Aktivität der Atemmuskulatur nicht-invasiv, d. h. an der Oberfläche, insbesondere Hautoberfläche, des Patienten. Damit ist in vorteilhafter Weise eine für den Patienten unangenehme oder schädliche invasive Messung der elektrischen Aktivität der Atemmuskulatur, beispielsweise in der Luftröhre invasiv mit einem EMG-Sensor, nicht mehr erforderlich. Der Beginn der Atemphase des Patienten und/oder die Eigenatemaktivität bzw. Eigenatmung des Patienten kann damit nicht-invasiv mit dem wenigstens einen sEMG-Sensor erfasst werden.

In einer ergänzenden Variante ist das Beatmungs- oder Anästhesiesystem mit einer Einrichtung, z. B. wenigstens eine Taste und/oder ein Hebel, zur Steuerung und/oder Regelung und/oder Veränderung des Anteils der Beatmung versehen. Ein Arzt kann damit den Anteil der Beatmung erhöhen und reduzieren und damit an den Gesundheitszustand des Patienten anpassen, beispielsweise für eine langsame Entwöhnung des Patienten von der Beatmung. Eine Erhöhung der Beatmung bedingt eine Reduzierung der Eigenatmung des Patienten und umgekehrt.

Vorzugsweise umfasst das Beatmungs- oder Anästhesiesystem wenigstens zwei sEMG-Sensoren, insbesondere wenigstens ein Paar an sEMG-Sensoren.

Vorzugsweise ist der wenigstens eine sEMG-Sensor in einem Abstand von weniger als 100 cm, 70 cm, 50 cm, 30 cm, 20 cm oder 10 cm zu dem Messmodul angeordnet.

In einer weiteren Ausgestaltung ist der wenigstens eine sEMG-Sensor mit einem Analogkabel oder mit einer Funkverbindung mit dem wenigstens einen Messmodul verbunden. Die von dem wenigstens einen sEMG-Sensor, vorzugsweise an der Hautoberfläche gemessene, Spannung aus der Atemmuskulaturaktivität wird mit dem Analogkabel zu dem wenigstens einen Messmodul geleitet. Dabei werden sehr kleine Spannungen im Bereich von einigen bis ungefähr 100 µV von dem wenigstens einen sEMG-Sensor gemessen.

Damit kann in dem wenigstens einen SEMG-Sensor die gemessene Spannung in digitale Signale umgewandelt werden und in das Messmodul und/oder die Haupteinheit übertragen werden.

In einer Variante ist das wenigstens eine Messmodul mit einer Analog-Digital-Wandeleinheit zur Umwandlung der von dem wenigstens einen sEMG-Sensor erfassten analogen elektromyographischen Muskelaktivität in digitale Signale versehen. Der wenigstens eine sEMG-Sensor erfasst nur sehr kleine Spannungen aus der Atemmuskulatur, so dass bei einer Leitung dieser sehr kleinen Ströme über eine größere Entfernung zu der Haupteinheit diese sehr kleinen Spannungen verfälscht werden können. Aus diesem Grund ist eine Umwandlung dieser sehr kleinen gemessenen Spannungen in digitale Signale sinnvoll und wirkungsvoll bei einer örtlich nahen Umwandlung in der Analog-Digital-Wandeleinheit in dem wenigstens einen Messmodul.

Zweckmäßig weist das wenigstens eine Messmodul eine Datenverarbeitungseinheit und/oder eine Energieversorgungseinheit auf.

In einer Variante ist die Energieversorgungseinheit wenigstens eine Batterie, vorzugsweise mehrere Batterien, und/oder ein Kondensator, vorzugsweise mehrere Kondensatoren, als Energiespeicher.

In einer weiteren Ausgestaltung ist die Haupteinheit mit einer Ladungseinheit für den wenigstens einen Energiespeicher versehen.

In einer ergänzenden Ausführungsform ist die Energieversorgungseinheit in dem wenigstens einen Messmodul wenigstens ein Stromkabel und das ' wenigstens eine Stromkabel ist zu der Haupteinheit geführt zum Leiten von elektrischem Strom von der Haupteinheit zu dem wenigstens einen Messmodul und vorzugsweise ist an der Haupteinheit der von dem wenigstens einen Stromkabel geleitete Strom mit einer Induktionseinheit mit zwei Spulen mittels Induktion von der Haupteinheit zu dem wenigstens einen Stromkabel leitbar. Der Strom wird in der Induktionseinheit von einer Primärspule mittels Induktion auf eine Sekundärspule übertragen und damit ist das Messmodul elektrisch und/oder magnetisch von der Haupteinheit entkoppelt.

Insbesondere ist die Induktionseinheit in oder an der Haupteinheit angeordnet.

Erfindungsgemäß ist zur Energieversorgung des wenigstens einen Messmoduls das Beatmungs- oder Anästhesiesystem mit wenigstens zwei Energiespeichern, insbesondere eine Batterie oder ein Kondensator, und einer Ladungs- und Schalteinheit versehen, sodass während einer Entladephase wenigstens einen Energiespeichers der wenigstens eine Energiespeicher von einer Energieversorgung der Haupteinheit elektrisch getrennt sowie mit dem Messmodul elektrisch verbunden ist und während einer Aufladephase des wenigstens einen Energiespeichers der wenigstens eine Energiespeicher elektrisch von dem wenigstens einem Messmodul getrennt ist sowie mit der Haupteinheit elektrisch verbunden ist. Damit ist das wenigstens eine Messmodul von der Haupteinheit elektrisch und/oder magnetisch entkoppelt, weil das wenigstens eine Messmodul nicht gleichzeitig mit einem elektrischen Kabel direkt mit der Haupteinheit verbunden ist.

In einer zusätzlichen Ausgestaltung ist das wenigstens eine Messmodul mit wenigstens einem MMG-Sensor (mechanomyographischer Sensor) und/oder wenigstens einem Beschleunigungssensor und/oder einem Mikrofon zur nicht-invasiven Erfassung der Muskelaktivität der Atemmuskulatur, so dass diese Sensoren nicht-invasive Sensoren sind. Der wenigstens eine MMG-Sensor (mechanomyographischer Sensor) und/oder der wenigstens einem Beschleunigungssensor und/oder das Mikrofon ist in analoger Weise wie der wenigstens eine sEMG-Sensor mit einem Analogkabel mit dem Messmodul verbunden.

Vorzugsweise ist von der Steuereinrichtung die Leistung der Gasfördereinrichtung und/oder der Druck des Inspirationsgases in Abhängigkeit von den Daten und/oder Strömen des wenigstens einem MMG-Sensors und/oder des wenigstens einen Beschleunigungssensors und/oder des Mikrofons steuerbar und/oder regelbar.

Erfindungsgemäßes Verfahren zur Steuerung und/oder Regelung eines Beatmungs- oder Anästhesiesystems, insbesondere eines in dieser Schutzrechtsanmeldung beschriebenen Beatmungs- oder Anästhesiesystems, mit den Schritten: Fördern von Atemluft durch wenigstens eine Gasleitung eines Atemluftleitungssystems mit einer Gasfördereinrichtung, Erfassen der elektromyographischen Muskelaktivität der Atemmuskulatur eines zu beatmenden Patienten vorzugsweise mit einem EMG-Sensor, Steuern und/oder Regeln der Leistung der Gasfördereinrichtung und/oder des Druckes der Inspirationsluft in Abhängigkeit von der erfassten Muskelaktivität der Atemmuskulatur, wobei die elektromyographische Muskelaktivität von wenigstens einem sEMG-Sensor an einer Hautoberfläche des zu beatmenden Patienten erfasst wird.

In einer zusätzlichen Ausgestaltung werden mit einer Analog-Digital-Wandeleinheit die von dem wenigstens einen sEMG-Sensor erfasste analoge elektromyographische Muskelaktivität in digitale Signale umgewandelt. Der sEMG-Sensor erfasst als analoge Muskelaktivität eine sehr kleine Spannung des Atemmuskels.

Zweckmäßig wird wenigstens ein Messmodul mit dem wenigstens einen sEMG-Sensor mit elektrischer Energie aus einer Haupteinheit des Beatmungs-oder Anästhesiergerätes versorgt.

In einer weiteren Ausgestaltung wird die elektrische Energie durch wenigstens ein Stromkabel von der Haupteinheit zu dem wenigstens einem Messmodul geleitet und dabei wird, vorzugsweise an der Haupteinheit, der Strom mit Induktion übertragen zur Verringerung der Koppelkapazität zwischen dem wenigstens einem Messmodul und der Haupteinheit (das wenigstens eine Messmodul ist damit elektrisch und/oder magnetisch von der Haupteinheit entkoppelt, weil eine Stromleitung von zu der Haupteinheit zu dem wenigstens einen Messmodul nur mittels Induktion möglich ist) und/oder an dem wenigstens einem Messmodul ist wenigstens ein Energiespeicher angeordnet und nach dem Erfassen der elektromyographischen Muskelaktivität mit dem wenigstens einem Messmodul an dem Patienten wird das wenigstens eine Messmodul zu der Haupteinheit gebracht und an der Haupteinheit wird der wenigstens eine Energiespeicher aufgeladen und vorzugsweise wird wenigstens ein anderes Messmodul nach dem Aufladen des wenigstens einen Energiespeichers an der Haupteinheit das wenigstens eine andere Messmodul zum Patienten gebracht zum Erfassen der elektromyographischen Muskelaktivität (das wenigstens eine Messmodul ist damit elektrisch und/oder magnetisch von der Haupteinheit entkoppelt, weil der Energiespeicher nur aufgeladen wird, wenn der Energiespeicher nicht mit dem Messmodul, sondern nur mit der Haupteinheit elektrisch verbunden ist) und/oder das wenigstens eine Messmodul wird mit wenigstens zwei Energiespeichern mit elektrischer Energie versorgt und während einer Entladephase wenigstens eines Energiespeichers wird das wenigstens eine Messmodul mit elektrischer Energie versorgt und während der Entladephase der wenigstens eine Energiespeicher elektrisch von der Haupteinheit getrennt wird und/oder während einer Aufladephase des wenigstens einen Energiespeichers der wenigstens eine Energiespeicher mit elektrischer Energie aus der Haupteinheit aufgeladen wird und während der Aufladephase der wenigstens eine Energiespeicher elektrisch von dem wenigstens einen Messmodul getrennt wird und/oder ist (das wenigstens eine Messmodul ist damit elektrisch und/oder magnetisch von der Haupteinheit entkoppelt, weil der Energiespeicher nur aufgeladen wird, wenn der Energiespeicher nicht mit dem Messmodul, sondern nur mit der Haupteinheit elektrisch verbunden ist).

In einer weiteren Ausgestaltung werden die digitalen Daten zur Muskelaktivität der Atemmuskulatur drahtlos (mit einer Funkverbindung) oder drahtgebunden von dem wenigstens einen Messmodul zu der Haupteinheit übertragen.

In einer weiteren Ausgestaltung ist eine Koppelkapazität zwischen dem wenigstens einem Messmodul und der Haupteinheit kleiner als 40 bis 50 pF, vorzugsweise kleiner als 30 bis 40 pF, insbesondere kleiner als 10 bis 12 pF (pFarad).

In einer ergänzenden Variante weist die Haupteinheit des Beatmungs- oder Anästhesiesystems einen CO₂-Absorber auf. Von dem CO₂-Absorber wird dem von dem zu beatmenden Patienten ausgeatmete Exspirationsgas das Kohlendioxid entzogen.

Zweckmäßig umfasst das Beamtungs- oder Anästhesiesystem einen Inspirations- und ein Exspirationsschlauch.

In einer weiteren Variante weist das Beatmungs- oder Anästhesiesystem ein Y-Stück oder eine Atemmaske auf.

In einer ergänzenden Variante sind der Inspirations- und/oder Exspirationsschlauch an dem Y-Stück oder der Atemmaske angeschlossen.

Vorzugsweise sind an dem Inspirations- und Exspirationsschlauch jeweils ein Rückschlagventil angeordnet.

In einer zusätzlichen Ausgestaltung weist die Haupteinheit des Beatmungs-oder Anästhesiesystem einen Anästhesiemittelreflektor auf. Mittels des Anästhesiemittelreflektors wird das Anästhesiemittel, z. B. Halotan oder Enfluran, dem Exspirationsgas entzogen und anschließend das entzogene Anästhesiemittel dem Inspirationsgas zugeführt.

In einer zusätzlichen Ausgestaltung weist die Haupteinheit des Beatmungs-oder Anästhesiesystems einen Anästhesiemitteldosierer mit einem Anästhesiemittelvorratsbehälter auf. Mittels des Anästhesiemitteldosierers wird dem Inspirationsgas Anästhesiemittel zugesetzt. Das Anästhesiemittel liegt dabei im Allgemeinen in einer flüssigen Form vor und das Anästhesiemittel wird in dem Anästhesiemitteldosierer von der flüssigen in die gasförmige Phase umgewandelt und somit dem Patienten zugeführt.

Bei der Ausbildung eines Atemluftkreissystems wird das von dem Patienten ausgeatmete Exspirationsgas wenigstens teilweise wieder als Inspirationsgas dem Patienten zugeführt. Dabei wird dem ausgeatmeten Exspirationsgas, vorzugsweise in dem CO₂-Absorber, das Kohlendioxid entzogen und vorzugsweise wird in einem Gasmischer das dem Patienten zuzuführende Inspirationsgas mit einer Mischung aus Sauerstoff, Lachgas, Luft und/oder Xenon angereichert.

Im Nachfolgenden werden Ausführungsbeispiele der Erfindung unter Bezugnahme auf die beigefügte Zeichnung näher beschrieben.

Es zeigen:
- Fig. 1: eine stark vereinfachte Darstellung eines Beatmungs- oder Anästhesiesystems in einem ersten Ausführungsbeispiel,
- Fig. 2: eine stark vereinfachte Darstellung des Beatmungs- oder Anästhesiesystems in einem zweiten Ausführungsbeispiel,
- Fig. 3: eine stark vereinfachte Darstellung des Beatmungs- oder Anästhesiesystems in einem dritten Ausführungsbeispiel,
- Fig. 4: einen Parkhalter in einem ersten Ausführungsbeispiel des Beatmungs- oder Anästhesiesystems gemäß Fig. 3,
- Fig. 5: den Parkhalter in einem zweiten Ausführungsbeispiel des Beatmungs- oder Anästhesiesystems gemäß Fig. 3,
- Fig. 6: eine Ladungs- und Schalteinheit des Beatmungs- und Anästhesiegerätes gemäß Fig. 7 und
- Fig. 7: eine stark vereinfachte Darstellung des Beatmungs- oder Anästhesiesystems in einem vierten Ausführungsbeispiel.

Beatmungsgeräte bzw. Beatmungssysteme 1 werden zur Beatmung von Patienten 20 und Anästhesiegeräte bzw. Anästhesiesysteme 1 werden neben der Beatmung auch zur Narkose von Patienten 20 eingesetzt. Das Beatmungs- oder Anästhesiesystem 1 weist dabei einen Atemluftkreissystem auf, d. h., dass das von dem Patienten 20 ausgeatmete Exspirationsgas für eine Rückatmung als Inspirationsgas wiederverwendet wird. Die Atemluft wird dabei von einer Gasfördereinrichtung 3 als Gebläse in einer Haupteinheit 2 durch Gasleitungen 4 in einem Atemluftkreissystem geleitet. Die Gasleitungen 4 sind somit an die Haupteinheit 2 angeschlossen. In den Gasleitungen 4 sind ein erstes, inspiratorisches Rückschlagventil und ein zweites expiratorisches Rückschlagventil angeordnet. Dadurch bildet sich eine Exspirationsgasleitung bzw. ein Exspirationsschlauch 8 und eine Inspirationsgasleitung bzw. ein Inspirationsschlauch 7 aus. Am Ende der Inspirations- und Exspirationsgasleitung 7, 8 ist ein Y-Stück angeschlossen, das mittels eines Patientenanschlussstückes das Inspirations- und Exspirationsgas zu und von einem zu beatmenden Patienten 20 leitet. Ein nicht dargestellter CO₂-Absorber in der Haupteinheit 2 absorbiert das in dem Exspirationsgas enthaltene Kohlendioxid. Ferner wird mit einem nicht dargestellten Anästhesiemittelreflektor in der Haupteinheit 2 und einem Anästhesiemitteldosierer das Inspirationsgas mit Anästhesiemittel angereichert (nicht dargestellt). Zusätzlich wird mittels eines nicht dargestellten Gasmischers in der Haupteinheit dem Inspirationsgas eine Mischung aus Sauerstoff und Lachgas zugeführt. Dem Gasmischer wird dabei mittels zweier Ventile separat der Sauerstoff und das Lachgas zugeführt.

Das Beatmungs- oder Anästhesiesystem 1 umfasst ferner ein Messmodul 5 mit zwei sEMG-Sensoren 6, wobei mit einem Analogkabel von geringer Länge, beispielsweise im Bereich zwischen 3 und 15 cm, die sEMG-Sensoren 6 elektrisch mit einem Messmodul 5 verbunden sind. Die beiden sEMG-Sensoren 6 liegen im Brustbereich auf der Haut des Patienten 20 auf, d. h. von den sEMG-Sensoren 6 wird die elektrische Aktivität der Atemmuskulatur nicht-invasiv gemessen. Das Messmodul 5 verfügt über nicht dargestellte Steckverbinder zum Anschließen von bis zu fünf Paaren an sEMG-Sensoren 6 (nicht dargestellt). Innerhalb eines Gehäuses (nicht dargestellt) des Messmoduls 5 ist eine Analog-Digital-Wandeleinheit 10 und eine Datenverarbeitungseinheit 11 angerordnet. Die sEMG-Sensoren 6 messen sehr kleine Ströme mit einer Spannung zwischen einigen bis ungefähr 100 µV, so dass bei einer Leitung dieser geringen Ströme über eine größere Entfernung durch ein langes Analogkabel eine Verfälschung dieser kleinen gemessenen Spannungen eintreten kann. Aus diesem Grund werden mit der Analog-Digital-Wandeleinheit 10 die von den beiden sEMG-Sensoren 6 erfassten Spannungen in einer geringen Entfernung von den sEMG-Sensoren 6 in digitale Signale umgewandelt. Das Messmodul 5 wird durch zwei Stromkabel 15 (nur ein Stromkabel 15 dargestellt) mit elektrischen Strom aus der Haupteinheit 2 versorgt. Die Stromkabel 15 in dem Messmodul 5 stellen damit eine Energieversorgungseinheit (nicht dargestellt) dar. In der Haupteinheit 2 ist ein DC-DC-Wandler 17 als Induktionseinheit 19 angeordnet mit einer nicht dargestellten Primär- und Sekundärspule. Dabei wird Gleichstrom zunächst moduliert, anschließend der modulierte Gleichstrom mittels Induktion von der Primär- auf die Sekundärspule übertragen und anschließend der in der Sekundärspule induzierte Strom wieder in Gleichstrom umgewandelt und durch die beiden Stromkabel 15 zu dem Messmodul 2 geleitet zur Stromversorgung des Messmoduls 2. Damit ist die Stromversorgung des Messmoduls 2 elektrisch bzw. magnetisch von der Haupteinheit 2 entkoppelt, so dass Störungen bei der Wandlung der gemessenen analogen Ströme in digitale Signale in der Analog-Digital-Wandeleinheit 10 sehr gering ausfallen.

Das Messmodul 5 ist mit einem Datenkabel 16 mit der Haupteinheit 2 verbunden zur Übertragung der von der Analog-Digital-Wandeleinheit 10 erzeugten digitalen Daten in die Haupteinheit 2. Dabei ist das Datenkabel 16 an eine Potentialtrenneinheit 18 in der Haupteinheit 2 angeschlossenen und von der Potentialtrenneinheit 18 werden die digitalen Daten zu einer Steuereinrichtung 9 in der Haupteinheit 2 übertragen. Das Datenkabel 16 kann ein elektrisches Kabel zur Leitung von Strom oder ein Glasfaserkabel zur optischen Übertragung von Daten sein. Mit der Potentialtrenneinheit 18 wird das elektrische Potential des Messmoduls 5 von dem elektrischen Potential der Haupteinheit 2 getrennt, damit auch bei unterschiedlichen elektrischen Potentialen in der Haupteinheit 2 und dem Messmodul 5 durch das Datenkabel 16 kein hieraus resultierender Strom fließt. In der Steuereinrichtung 9 werden die digitalen Signale ausgewertet und in Abhängigkeit von den digitalen Signalen, d. h. der von den beiden sEMG-Sensoren 6 auf der Hautoberfläche gemessenen elektrischen Potentialdifferenz aus der Atemmuskelaktivität, die Leistung der Gasfördereinrichtung 3 gesteuert und/oder geregelt.

In der Datenverarbeitungseinheit 11 können digitale Signale gespeichert und verarbeitet werden und ferner kann ein Messmodul 5 und damit auch ein Patient 20 von der Steuerungseinheit 9 identifiziert werden bei einer Verwendung von mehreren Messmodulen 5 für die gleiche Haupteinheit 2. Außerdem können von der Datenverarbeitungseinheit 11 Checksummen der übertragenen digitalen Daten und/oder Kontrollsignale zu der Steuereinrichtung 9 übertragen werden, um mögliche Fehler bei der Übertragung von dem Messmodul 5 zu der Haupteinheit 2 bzw. der Steuereinrichtung 9 zu erfassen. Damit soll eine falsche Beatmung des Patienten 20 aufgrund von Datenübertragungsfehlern ausgeschlossen werden.

Das Messmodul 5 weist außerdem'wenigstens einen Sensor (nicht dargestellt) zur Erfassung eines Patientengrounds, d. h. des durchschnittlichen elektrischen Potentials des Patienten 20, auf. Abweichend hiervon kann der Patientenground auch aus dem von den sEMG-Sensoren 6 erfassten durchschnittlichen elektrischen Potential berechnet werden. Es handelt sich bei diesem Sensor um einen nicht-invasiven Sensor, der das elektrische Potential an der Hautoberfläche misst. Das elektrische Potential in dem Messmodul 5 wird mit der Datenverarbeitungseinheit 11 derart gesteuert, dass das elektrische Potential in dem Messmodul 5 im Wesentlichen dem Patientenground entspricht, um hieraus resultierende Ströme in dem Analogkabel zur Verbindung der sEMG-Sensoren 6 mit dem Messmodul 5 zu verhindern.

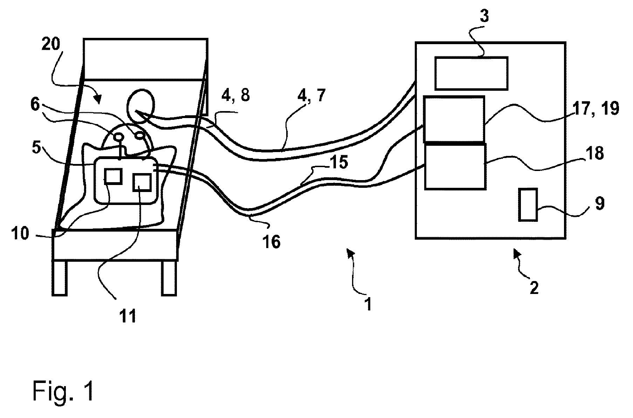

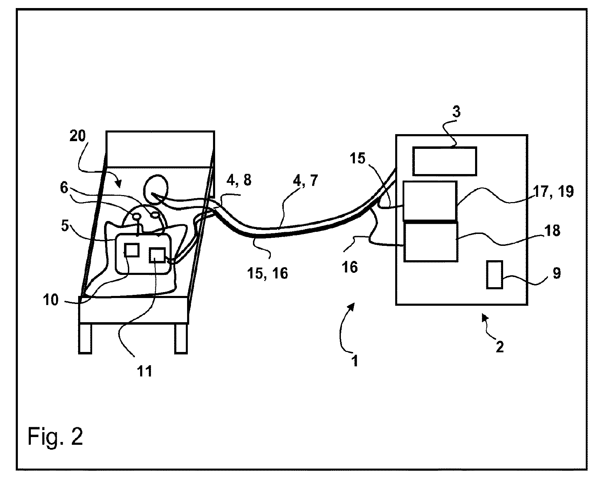

In Fig. 2 ist ein zweites Ausführungsbeispiel des Beatmungs- oder Anästhesiesystems 1 dargestellt. Im Nachfolgenden werden im Wesentlichen nur die Unterschiede zu dem ersten Ausführungsbeispiel gemäß Fig. 1 beschrieben. Das Stromkabel 15 und das Datenkabel 16 zur Verbindung des Messmoduls 5 mit der Haupteinheit 2 ist in den Inspirationsschlauch 7 und/oder den Exspirationsschlauch 8 integriert, so dass die Haupteinheit 2 und das Messmodul 5 nur mit eine Leitungseinheit bzw. einem Kabelkanal miteinander verbunden sind sowohl zur pneumatischen als auch zur elektrischen Verbindung.

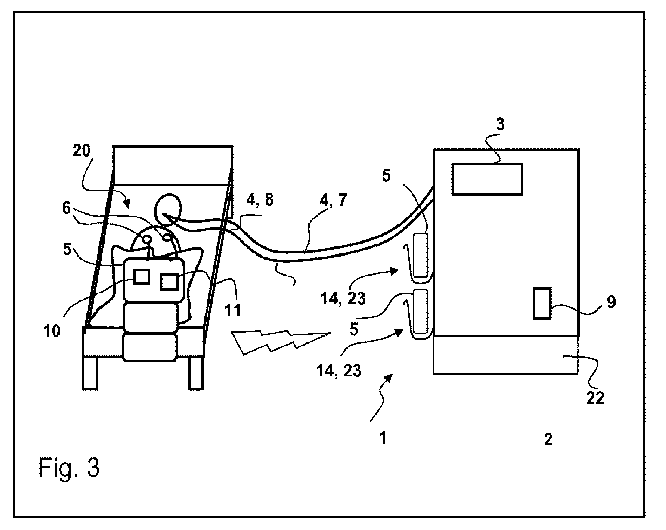

In Fig. 3 ist ein drittes Ausführungsbeispiel des Beatmungs- oder Anästhesiesystems 1 dargestellt. Im Nachfolgenden werden im Wesentlichen nur die Unterschiede zu dem ersten Ausführungsbeispiel gemäß Fig. 1 beschrieben. In das Messmodul 5 ist eine Batterie 13 als Energiespeicher 12 eingebaut und dient zur elektrischen Energieversorgung des Messmoduls 5. Außerdem ist in das Messmodul 5 ein Sender 21 und in die Haupteinheit 2 ein Empfänger 22 eingebaut zur drahtlosen Übertragung der digitalen Signale von dem Messmodul 5 zu der Haupteinheit 2 bzw. der Steuereinrichtung 9. Von dem Empfänger 22 werden die digitalen Signale bezüglich der elektromyographischen Atemmuskelaktivität zu der Steuereinrichtung 9 übertragen. Dabei kann von der Steuereinrichtung 9 ein Messmodul 5 identifiziert werden mit einer bidirektionalen Funkverbindung.

Das Beatmungs- oder Anästhesiesystem 1 umfasst zwei oder drei Messmodule 5, weil sich ein Messmodul 5 beim Patienten 5 befindet zur Erfassung der elektromyographischen Atemmuskelaktivität und Übertragung der digitalen Daten, so dass die Batterie 13 in dem Messmodul 5 am Patienten 5 entladen wird. Das zweite oder dritte Messmodul 5 ist in einer Ladungseinheit 14 als Parkhalter 23 angeordnet und wird aufgeladen. Das Messmodul 5 an dem Patienten 20 wird bei einem Unterschreiten eines vorgegebenen Ladezustandes der Batterie 13 durch ein in der Ladungseinheit 14 aufgeladenes Messmodul 5 ersetzt. Dabei ist das Messmodul 5 mit einer nicht dargestellten zentralen Steckeinheit versehen, mit dem sämtliche an dem Patienten 20 angeordnete sEMG-Sensoren 6 mit dem Messmodul 5 verbunden werden können. Damit besteht auch keine direkte elektrische Verbindung zwischen der Haupteinheit 2 und dem Messmodul 5 am Patienten 20, so dass damit das Messmodul 5 an dem Patienten 20 elektrisch und magnetisch von der Haupteinheit 2 entkoppelt ist. Die Aufladezeit eines Messmoduls 5 in dem Parkhalter 23 beträgt maximal die Hälfte der Betriebsdauer des Messmoduls 5 am Patienten. Zweckmäßig beträgt die Aufladezeit 1/4 oder 1/6 der Betriebsdauer. Eine Betriebsdauer von 24 h steht z. B. eine Aufladezeit von 4 h gegenüber. Während der Aufladens des Messmoduls 5 können auch digitale Daten mittels nicht dargestellter Kontaktelemente von dem Messmodul 5 in die Steuereinrichtung 9 übertragen werden, beispielsweise bezüglich der Historie des Messmoduls 5, der Ladezyklen, der Betriebsstunden und Statusinformationen. Das Messmodul 5 kann von der Steuereinrichtung 9 auch identifiziert werden mittels Daten in der Datenverarbeitungseinheit 11.

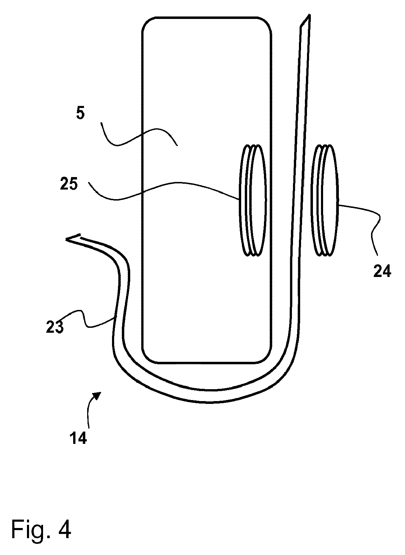

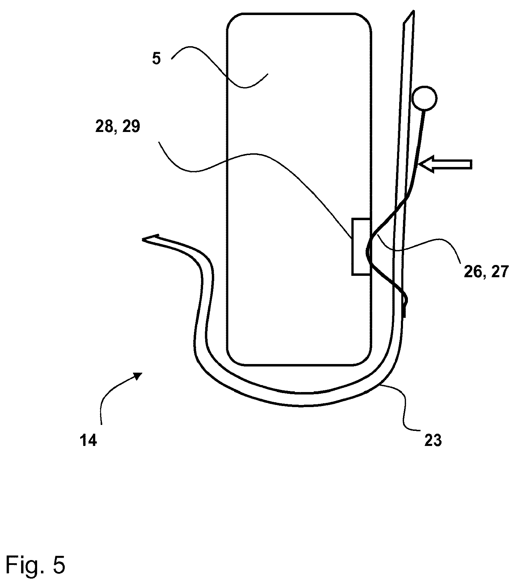

Fig. 4 und 5 zeigen zwei Ausführungsbeispiele für die Ladungseinheit 14. Im ersten Ausführungsbeispiel gemäß Fig. 1 erfolgt die Energie- bzw. Stromübertragung von einer Geberspule 24 an der Haupteinheit 2 mittels elektrischer Induktion kontaktlos zu einer Nehmerspule 25 in dem Messmodul 5. Das zweite Ausführungsbeispiel (Fig. 5) der Ladungseinheit 14 zeigt eine kontaktgebundene Stromübertragung von der Haupteinheit 2 mit einem als Metallfeder 27 ausgebildeten Kontaktelemerit 26 an der Haupteinheit 2 und einem Metallplättchen 29 als Gegenkontaktelement 28. Die Ladungseinheit 2 weist je zwei Kontaktelemente 26 und das Messmodul 5 ist mit zwei
Gegenkontaktelementen 28 versehen. Damit kann elektrischer Strom von der Haupteinheit 2 in die Batterie 13 geleitet werden.

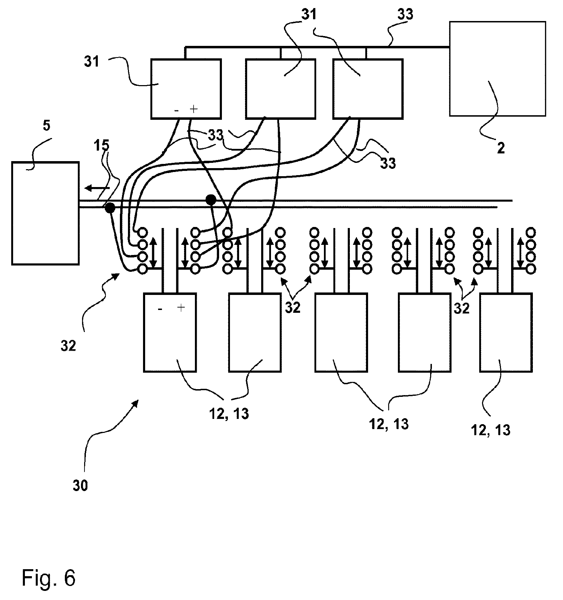

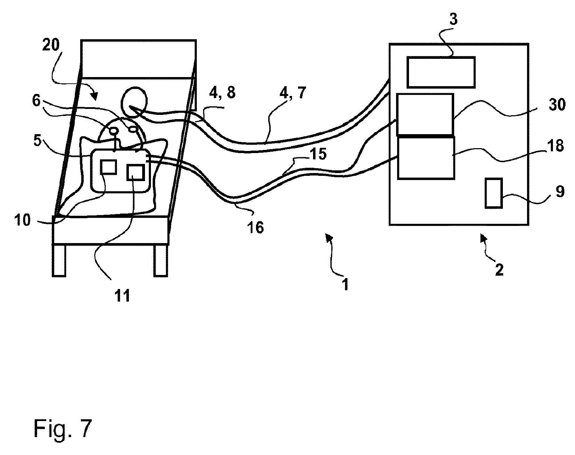

In Fig. 6 und 7 ist ein viertes Ausführungsbeispiel des Beatmungs- oder Anästhesiesystems 1 dargestellt. Im Nachfolgenden werden im Wesentlichen nur die Unterschiede zu dem ersten Ausführungsbeispiel gemäß Fig. 1 beschrieben. In der Haupteinheit 2 ist eine Ladungs- und Schaleinheit 30 angeordnet. In der Ladungs- und Schalteinheit 30 werden Batterien 13 mit Ladungsteilen 31 aufgeladen. Die Ladungsteile 31 sind mit Ladungskabel 33 mit der Haupteinheit 2 und den Batterien 13 verbunden. Außerdem sind zu den Batterien 13 die Stromkabel 15 geführt, mit denen der Strom aus den Batterien 13 in das Messmodul 5 geleitet wird. Schalteinheiten 32 an den Batterien 13 dienen dazu, dass nur eine Batterie 13 mit dem Messmodul 5 verbunden ist zur Stromversorgung des Messmoduls 5 mit der einen Batterie 13 und wenigstens eine andere Batterie 13 nur mit einem Ladungsteil 31 verbunden ist zum Aufladen der wenigstens einen anderen Batterie 13. Nach dem Entladen einer Batterie 13 wird die entladene Batterie 13 mit der Schalteinheit 32 von dem Messmodul 5 elektrisch getrennt und eine andere aufgeladene Batterie 13 mit dem Messmodul 5 elektrisch verbunden. Die entladene Batterie 13 wird anschließend mit dem Ladungsteil 31 elektrisch verbunden und aufgeladen. Die Batterien 13 sind damit niemals zeitglich sowohl mit dem Messmodul 5 als auch mit dem Ladungsteil 31 bzw. der Haupteinheit 2 elektrisch verbunden, so dass das Messmodul 5 elektrisch bzw. magnetisch von der Haupteinheit 2 entkoppelt ist. Die Ladungs-und Schalteinheit 30 kann anstelle in der Haupteinheit 2 auch in dem Messmodul 5 angeordnet sein (nicht dargestellt).

Insgesamt betrachtet sind mit dem erfindungsgemäßen Beatmungs- oder Anästhesiesystem 1 wesentliche Vorteile verbunden. Die Gasfördereinrichtung 3 wird in Abhängigkeit von der elektromyographischen Atemmuskelaktivität betrieben, welche mit sEMG-Sensoren 6 gemessen wird, so dass eine nachteilige invasive Messung nicht mehr erforderlich ist. Die Energieversorgung des Messmoduls 5 ist elektrisch und magnetisch von der Haupteinheit 2 entkoppelt, so dass keine Störungen wegen einer elektrischen und magnetischen Koppelung des Messmoduls 5 mit der Haupteinheit 2 auftreten.

### BEZUGSZEICHENLISTE

- 1: Beatmungs- oder Anästhesiesystem
- 2: Haupteinheit
- 3: Gasfördereinrichtung
- 4: Gasleitung
- 5: Messmodul
- 6: sEMG-Sensor
- 7: Inspirationsschlauch
- 8: Exspirationsschlauch
- 9: Steuereinrichtung
- 10: Analog-Digital-Wandeleinheit
- 11: Datenverarbeitungseinheit
- 12: Energiespeicher
- 13: Batterie
- 14: Ladungseinheit
- 15: Stromkabel
- 16: Datenkabel
- 17: DC-DC-Wandler
- 18: Potentialtrenneinheit
- 19: Induktionseinheit
- 20: Patient
- 21: Sender
- 22: Empfänger
- 23: Parkhalter
- 24: Geberspule
- 25: Nehmerspule
- 26: Kontaktelement
- 27: Metallfeder
- 28: Gegenkontaktelement
- 29: Metallplättchen
- 30: Ladungs- und Schalteinheit
- 31: Ladungsteil
- 32: Schalteinheit
- 33: Ladungskabel

## Patentansprüche

1. Beatmungs- oder Anästhesiesystem zur Beatmung eines Patienten, umfassend
- eine Gasfördereinrichtung (3),
- wenigstens eine Gasleitung (4) zur Ausbildung eines Atemluftleitungssystems,
- wenigstens einen sEMG-Sensor (6) zur nicht-invasiven Erfassung der elektromyographischen Muskelaktivität der Atemmuskulatur eines zu beatmenden Patienten (20) an dessen Hautoberfläche,
- eine Steuereinrichtung (9) zur Steuerung und/oder Regelung der Leistung der Gasfördereinrichtung (3) in Abhängigkeit von der erfassten Muskelaktivität der Atemmuskulatur,
- eine Haupteinheit (2) mit einem Hauptgehäuse und der Gasfördereinrichtung (3),
- wenigstens ein Messmodul (5), an welchem der wenigstens eine EMG-Sensor (6) angeordnet ist, wobei das wenigstens eine Messmodul (5) eine von der Haupteinheit (2) getrennte Baueinheit ist,
**dadurch gekennzeichnet,**
**dass** die Haupteinheit (2) eine Ladungs- und Schalteinheit (30) umfasst, wobei in der Ladungs- und Schalteinheit Batterien (13) mittels Ladungsteilen (31) aufgeladen werden,
**dass** die Ladungs- und Schalteinheit (30) wiederum Schalteinheiten umfasst, welche dazu ausgebildet sind,
- nur eine erste Batterie mit dem Messmodul (5) zur Stromversorgung des Messmoduls zu verbinden,
- ferner wenigstens eine andere Batterie nur mit einem Ladungsteil (31) zum Aufladen der anderen Batterie zu verbinden,
- und ferner die erste Batterie nach dem Entladen von dem Messmodul elektrisch zu trennen und die andere, aufgeladene Batterie mit dem Messmodul elektrisch zu verbinden.

2. Beatmungs- oder Anästhesiesystem nach Anspruch 1, **dadurch gekennzeichnet, dass** das wenigstens eine Messmodul (5) mit einer Analog-Digital-Wandeleinheit (10) zur Umwandlung der von dem wenigstens einen sEMG-Sensor (6) erfassten analogen elektromyographischen Muskelaktivität in digitale Signale versehen ist.

3. Beatmungs- oder Anästhesiesystem nach einem oder mehreren der Ansprüche 1 bis 2, **dadurch gekennzeichnet, dass** das wenigstens eine Messmodul (5) eine Datenverarbeitungseinheit (11) und/oder eine Energieversorgungseinheit aufweist.

4. Beatmungs- oder Anästhesiesystem nach Anspruch 3 , **dadurch gekennzeichnet, dass** das wenigstens eine Messmodul (5) eine Energieversorgungseinheit aufweist, und dass die Energieversorgungseinheit wenigstens eine Batterie (13) und/oder ein Kondensator als Energiespeicher (12) ist.

5. Beatmungs- oder Anästhesiesystem nach Anspruch 4 , **dadurch gekennzeichnet, dass** die Haupteinheit (2) mit einer Ladungseinheit (14) für den wenigstens einen Energiespeicher (12) versehen ist.

6. Beatmungs- oder Anästhesiesystem nach Anspruch 5, **dadurch gekennzeichnet, dass** die Energieversorgungseinheit in dem wenigstens einen Messmodul (5) wenigstens ein Stromkabel (15) ist und das wenigstens eine Stromkabel (15) zu der Haupteinheit (2) geführt ist zum Leiten von elektrischem Strom von der Haupteinheit (2) zu dem wenigstens einen Messmodul (5) und vorzugsweise an der Haupteinheit (2) der von dem weinigstens einen Stromkabel (15) geleitete Strom mit einer Induktionseinheit (19) mit zwei Spulen mittels Induktion von der Haupteinheit (2) zu dem wenigstens einen Stromkabel (15) leitbar ist.

7. Beatmungs- oder Anästhesiesystem nach einem oder mehreren der vorhergehenden Ansprüche, **dadurch gekennzeichnet, dass** das wenigstens eine Messmodul (5) zur Erfassung der Muskelaktivität der Atemmuskulatur mit wenigstens einem MMG-Sensor oder wenigstens einem Beschleunigungssensor oder einem Mikrofon versehen ist.

## Claims

1. A ventilation or anaesthetic system for ventilating a patient, comprising
- a gas-conveying device (3),
- at least one gas line (4) to form a respiratory-air line system,
- at least one sEMG sensor (6) for non-invasive detection of the electromyographic muscle activity of the respiratory musculature of a patient (20), who is to be ventilated, at the surface of the patient's skin,
- a control device (9) to control and/or regulate the capacity of the gas-conveying device (3) as a function of the detected muscle activity of the respiratory musculature,
- a main unit (2) having a main housing and the gas-conveying device (3),
- at least one measuring module (5) at which the at least one EMG sensor (6) is arranged, wherein the at least one measuring module (5) is a structural unit that is separated from the main unit (2),
**characterised**
**in that** the main unit (2) comprises a charging and switching unit (30), wherein in the charging and switching unit batteries (13) are charged by means of charging portions (31),
**in that** the charging and switching unit (30) in turn comprises switching units which are formed
- to connect only one first battery to the measuring module (5) to supply current to the measuring module,
- to connect, furthermore, at least one other battery only to one charging portion (31) to charge the other battery,
- and, furthermore, to separate the first battery electrically from the measuring module after discharge and to connect the other charged battery electrically to the measuring module.

2. A ventilation or anaesthetic system according to claim 1, **characterised in that** the at least one measuring module (5) is provided with an analog-to-digital converter unit (10) to convert the analog electromyographic muscle activity, detected by the at least one sEMG sensor (6), into digital signals.

3. A ventilation or anaesthetic system according to one or more of claims 1 to 2, **characterised in that** the at least one measuring module (5) has a data-processing unit (11) and/or an energy supply unit.

4. A ventilation or anaesthetic system according to claim 3, **characterised in that** the at least one measuring module (5) has an energy supply unit, and **in that** the energy supply unit is at least one battery (13) and/or a capacitor as an energy store (12).

5. A ventilation or anaesthetic system according to claim 4, **characterised in that** the main unit (2) is provided with a charging unit (14) for the at least one energy store (12).

6. A ventilation or anaesthetic system according to claim 5, **characterised in that** the energy supply unit in the at least one measuring module (5) is at least one power cable (15), and the at least one power cable (15) is guided to the main unit (2) to conduct electric current from the main unit (2) to the at least one measuring module (5), and preferably at the main unit (2) the current conducted by the at least one power cable (15) can be conducted with an induction unit (19) with two coils by means of induction from the main unit (2) to the at least one power cable (15).

7. A ventilation or anaesthetic system according to one or more of the preceding claims, **characterised in that** the at least one measuring module (5) for the detection of the muscle activity of the respiratory musculature is provided with at least one MMG sensor or at least one acceleration sensor or a microphone.

## Revendications

1. Système de ventilation ou d'anesthésie pour la ventilation d'un patient, comprenant
- un dispositif de transport de gaz (3),
- au moins une conduite de gaz (4) pour réaliser un système de conduite d'air respiratoire,
- au moins un capteur sEMG (6) pour l'acquisition non invasive de l'activité musculaire électromyographique de la musculature respiratoire d'un patient (20) à ventiler à la surface de sa peau,
- un dispositif de commande (9) pour la commande et/ou la régulation du débit du dispositif de transport de gaz (3) en fonction de l'activité musculaire ou acquise de la musculature respiratoire,
- une unité principale (2) présentant un boîtier principal et le dispositif de transport de gaz (3),
- au moins un module de mesure (5) sur lequel ledit au moins un capteur EMG (6) est disposé, ledit au moins un module de mesure (5) étant une unité modulaire séparée de l'unité principale (2),
**caractérisé en ce**
**que** l'unité principale (2) comprend une unité de charge et de commutation (30), des batteries (13) étant chargées au moyen d'éléments de charge (31) dans l'unité de charge et de commutation (30),
**que** l'unité de charge et de commutation (30) comprend d'autre part des unités de commutation qui sont réalisées
- pour relier seulement une première batterie au module de mesure (5) pour l'alimentation électrique du module de mesure,
- pour relier en outre au moins une autre batterie à seulement un élément de charge (31) pour recharger ladite autre batterie,
- et en outre pour séparer électriquement la première batterie du module de mesure après sa décharge et relier électriquement l'autre batterie, rechargée, au module de mesure.

2. Système de ventilation ou d'anesthésie selon la revendication 1, **caractérisé en ce que** ledit au moins un module de mesure (5) est pourvu d'une unité de conversion analogique-numérique (10) pour convertir l'activité musculaire électromyographique analogique détectée par ledit au moins un capteur sEMG (6) en signaux numériques.

3. Système de ventilation ou d'anesthésie selon l'une ou plusieurs des revendications 1 à 2, **caractérisé en ce que** ledit au moins un module de mesure (5) présente une unité de traitement de données (11) et/ou une unité d'alimentation en énergie.

4. Système de ventilation ou d'anesthésie selon la revendication 3, **caractérisé en ce que** ledit au moins un module de mesure (5) présente une unité d'alimentation en énergie et que l'unité d'alimentation en énergie est au moins une batterie (13) et/ou un condensateur utilisé comme accumulateur d'énergie (12).

5. Système de ventilation ou d'anesthésie selon la revendication 4, **caractérisé en ce que** l'unité principale (2) est pourvue d'une unité de charge (14) pour ledit au moins un accumulateur d'énergie (12).

6. Système de ventilation ou d'anesthésie selon la revendication 5, **caractérisé en ce que** l'unité d'alimentation en énergie dans ledit au moins un module de mesure (5) est au moins un câble électrique (15) et ledit au moins un câble électrique (15) est amené à l'unité principale (2) pour conduire un courant électrique de l'unité principale (2) vers ledit au moins un module de mesure (5) et de préférence, sur l'unité principale (2), le courant conduit par ledit au moins un câble électrique (15) peut être transmis par induction de l'unité principale (2) audit au moins un câble électrique (15) au moyen d'une unité d'induction (19) à deux bobines.

7. Système de ventilation ou d'anesthésie selon l'une ou plusieurs des revendications précédentes, **caractérisé en ce que** ledit au moins un module de mesure (5) est pourvu d'au moins un capteur MMG ou d'au moins un capteur d'accélération ou d'un microphone pour détecter ou acquérir l'activité musculaire de la musculature respiratoire.
